# EUROPÄISCHE PATENTANMELDUNG

(11) **EP 0 819 440 A2**
(43) Veröffentlichungstag der Anmeldung: **21.01.1998**
(21) Anmeldenummer: 97111792.4
(22) Anmeldetag: 11.07.1997
(51) Int. Cl.: A61M 5/14

(54) **Infussionsset**

(30) Priorität: 19.07.1996 DE 29612534 U
(71) Anmelder: B. BRAUN MELSUNGEN AG, 34212 Melsungen (DE)
(72) Erfinder: Dammeyer, Rainer, D-34212 Melsungen (DE); Loretti, Maurice, 1219 Châtelaine, Genève (CH); Fuchs, Juergen, D-34308 Bad Emstal (DE)
(74) Vertreter: Selting, Günther, Dipl.-Ing.

(57) **Zusammenfassung**

Ein Infusionsset zur Applizierung von Infusionsflüssigkeiten besteht aus einem Container (11), der eine Entnahmevorrichtung (15) aufweist, und aus einer an die Entnahmevorrichtung (15) anschließbaren Übertragungsvorrichtung (12) mit einer Tropfkammer (27) und einem Übertragungsschlauch (12). Die Entnahmevorrichtung (15) des Containers (11) und die Übertragungsvorrichtung weisen zusammenpassende Kupplungselemente (20,21) auf, wobei eines der Kupplungselemente (20) einen Verschluß enthält, der beim Zusammenstecken mit dem anderen Kupplungselement (21) automatisch öffnet oder nach dem Zusammenstecken durch externe Betätigung manuell öffnet.

## Beschreibung

Die Erfindung betrifft ein Infusionsset mit einem die Infusionsflüssigkeit enthaltenden Container und einer an eine Entnahmevorrichtung des Containers anzuschließenden Übertragungsvorrichtung, die eine Tropfkammer und einen Überleitungsschlauch aufweist.

Üblicherweise ist bei Infusionen die Infusionsflüssigkeit in einem Container in Form einer Flasche oder eines Beutels enthalten. Der Container weist eine Entnahmevorrichtung in Form eines gummielastischen Stopfens auf, die den Container dicht verschließt. Zur Entnahme der Infusionsflüssigkeit wird eine Übertragungsvorrichtung benutzt, die an einer Tropfkammer einen Einstechdorn aufweist, welcher durch den Stopfen hindurch in den Container hineingestochen wird. Dann läuft die Flüssigkeit durch den Einstechdorn, die Tropfkammer und den Übertragungsschlauch zum Patienten.

Üblicherweise werden der die Infusionsflüssigkeit enthaltende Container und die Übertragungsvorrichtung separat geliefert. Die Übertragungsvorrichtung ist steril verpackt und wird nach dem Öffnen der Verpackung mit einem beliebigen Container verbunden. Hierbei muß der Einstechdorn mit Kraft durch den Verschlußstopfen des Containers hindurchgestochen werden, was eine gewisse Geschicklichkeit erfordert und häufig dazu führt, daß die Perforation nicht an der gewünschten Stelle erfolgt. Außerdem entsteht ein schwerwiegendes Sterilitätsproblem dadurch, daß bei der Manipulation der Einstechdorn kontaminiert werden kann. Dabei ist zu berücksichtigen, daß sowohl im Krankenhausbetrieb als auch insbesondere bei der Unfallversorgung von Patienten Infusionen häufig in größter Eile und in Streßsituationen durchgeführt werden müssen, so daß nicht garantiert werden kann, daß das Verbinden des Containers mit der Übertragungsvorrichtung stets ordnungsgemäß durchgeführt wird.

Der Erfindung liegt die Aufgabe zugrunde, ein Infusionsset zu schaffen, dessen Handhabung vereinfacht ist und das einen definierten Anschluß der Übertragungsvorrichtung an den Container unter sterilen Bedingungen erleichtert.

Die Lösung dieser Aufgabe erfolgt erfindungsgemäß mit den im Anspruch 1 angegebenen Merkmalen.

Bei dem erfindungsgemäßen Übertragungsgerät haben die Entnahmevorrichtung des Containers und die Übertragungsvorrichtung zusammenpassende Kupplungselemente, die nach Art einer Schlauchkupplung definiert zusammengesteckt werden können und eine geordnete und dichte Verbindung bewirken. Damit ist anstelle des bisher üblichen Hindurchstechens eines Einstechdorns durch einen Gummistopfen ein einfaches Zusammenstecken von Kupplungselementen möglich. Eines der Kupplungselemente, vorzugsweise dasjenige des Containers, enthält einen Verschluß, der beim Zusammenstecken mit dem anderen Kupplungselement automatisch öffnet oder nach dem Zusammenstecken durch externe Betätigung manuell öffnet. Damit ist sichergestellt, daß vor dem Zusammenstecken keine Keimwanderung in das betreffende Kupplungselement hinein und in die angeschlossene Vorrichtung erfolgen kann. Insbesondere wird aber erreicht, daß der Flüssigkeitsdurchfluß erst freigegeben wird, wenn die beiden Kupplungsteile ordnungsgemäß zusammengesteckt und so miteinander verbunden sind.

Der Begriff Infusionsset ist im Zusammenhang mit der vorliegenden Erfindung so zu verstehen, daß er für jegliche Zuführung von Flüssigkeit in den Körper eines Patienten geeignet ist. Er umfaßt also auch beispielsweise Transfusionssysteme. Der Container besteht vorzugsweise aus einem flexiblen Beutel, kann jedoch auch aus einer Flasche oder einem Blow-fill-Seal-Container (Bottlepack-Behälter) bestehen.

Das Kupplungssystem aus zusammenpassenden Kupplungselementen ist vorzugsweise als Luer-Lock-Kupplung ausgebildet. Hierbei besteht der Vorteil, daß die Kupplungselemente nicht nur durch Klemmung festgehalten, sondern auch durch Verschraubung verriegelt und gegen Herausziehen gesichert werden.

Das Kupplungselement der Übertragungsvorrichtung kann einteilig mit der Tropfkammer ausgebildet sein. Durch die Einteiligkeit wird die Handhabbarkeit der Kupplung verbessert. Ferner wird die Anzahl herzustellender Einzelteile und damit der Gesamtherstellungsaufwand verringert.

Bei einer Ausführungsform der Erfindung, die sich insbesondere für die Applikation von Cytostatika eignet, ist der Verschluß als selbstschließendes Ventil ausgebildet. Dies hat den Vorteil, daß die Verbindung der Kupplungselemente jederzeit gelöst werden kann, und daß dann der Flüssigkeitsfluß automatisch durch das Schließen des Ventils, das sich an der Entnahmevorrichtung des Containers befindet, unterbrochen wird. Ein Nachlaufen oder Nachtropfen wird dabei verhindert.

Alternativ hierzu kann das Ventil auch als manuell zu betätigendes Ventil, beispielsweise als Kükenventil, ausgebildet sein.

Bei einer vereinfachten Variante der Erfindung ist der Verschluß ein Abbrechteil. Dieses Abbrechteil kann entweder so ausgebildet sein, daß es nach dem Herstellen der Kupplungsverbindung manuell abgebrochen wird, oder in der Weise, daß es beim Zusammenstecken der Kupplungselemente automatisch abbricht.

Zweckmäßigerweise ist das Infusionsset aus Container und Übertragungsvorrichtung steril in einer gemeinsamen Umverpackung enthalten. Damit kann die erforderliche Keimfreiheit vor der Benutzung sichergestellt werden. Wenn der Verschluß derart ausgebildet ist, daß er manuell geöffnet werden muß, können die Kupplungselemente bereits in der Umverpackung zusammengesteckt sein.

Ein weiterer Aspekt besteht darin, daß ein abbrechbarer Verschluß zugleich ein Brechsiegel darstellt, das den erstmaligen Zugang zum Containerinhalt optisch erkennbar macht. Im Falle eines selbstschließenden Ventils kann die Funktion des Brechsiegels von einer Verschlußkappe übernommen werden, die auf das Kupplungselement des Containers aufgesetzt ist und deren erstmaliges Öffnen optisch erkennbar ist.

Im folgenden werden unter Bezugnahme auf die Zeichnungen Ausführungsbeispiele der Erfindung näher erläutert.

Es zeigen:
- Fig. 1: ein in einer Verpackung steril enthaltenes Infusionsset, bestehend aus Container und Übertragungsvorrichtung,
- Fig. 2: die Kupplungselemente von Container und Übertragungsvorrichtung im auseinandergenommenen Zustand,
- Fig. 3: eine Ausführungsform eines Infusionssets mit selbstschließendem Ventil,
- Fig. 4: einen Längsschnitt durch das Kupplungselement mit Ventil nach Fig. 3,
- Fig. 5: ein Ausführungsbeispiel, bei dem das eine Kupplungselement ein beim Zusammenstecken automatisch abbrechendes Abbrechteil enthält, und
- Fig. 6: die Kupplungsverbindung nach Fig. 5 im zusammengesteckten Zustand.

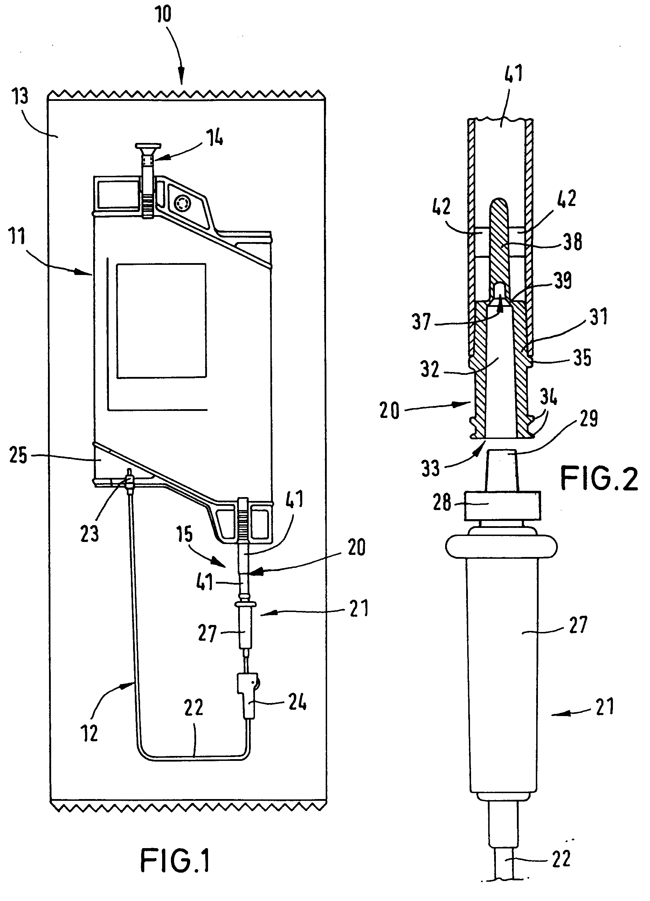

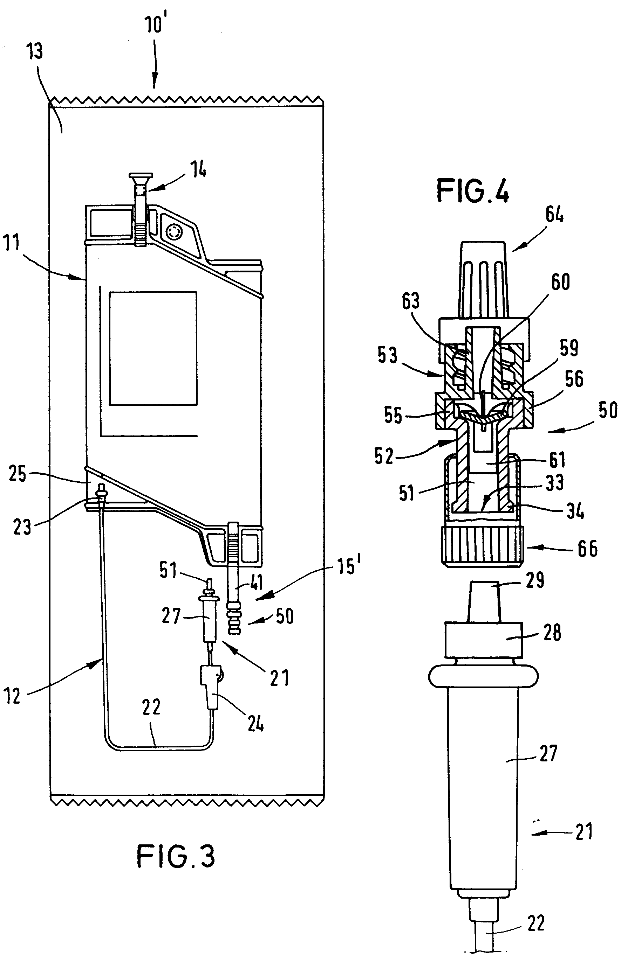

In den Fign. 1 und 3 ist jeweils ein Infusionsset 10,10' dargestellt, das als Container für eine Infusionslösung einen transparenten Kunststoffbeutel 11 aufweist, der zusammen mit einer Übertragungsvorrichtung 12 steril in einer transparenten Kunststoff-Umverpackung 13 enthalten ist. Die Übertragungsvorrichtung 12 dient dazu, den Infusionsbeutel 11 mit einem Infusionsgerät bzw. -pumpe und/oder direkt mit einem Patienten zu verbinden.

Der annähernd parallelogrammförmige Infusionsbeutel 11 weist in einem oberen Eckbereich ein verschlossenes Ventil 14 auf, das dem Befüllen des Infusionsbeutels 11 mit der Infusionslösung dient und danach dauerhaft verschlossen wird. In dem dem Ventil 14 gegenüberliegenden Eckbereich des Infusionsbeutels 11 ist als Entnahmevorrichtung 15,15' an einem Schlauchstück 41 ein Kupplungselement 20 angeordnet, das mit einem weiteren Kupplungselement 21 der Übertragungsvorrichtung 12 zusammensteckbar ist.

Die Übertragungsvorrichtung 12 weist einen Übertragungsschlauch 22 auf, an dessem einen Ende das Kupplungselement 21 befestigt ist. Am anderen Ende des Schlauches 22 ist ein patientenseitiges Kupplungsteil 23 zum Anschluß der Übertragungsvorrichtung 12 an eine Infusionsnadel oder eine Infusionspumpe angebracht. Das patientenseitige Kupplungsteil 23 ist an einer Lasche 25 des Infusionsbeutels 11 leicht lösbar angeschlagen.

An das Kupplungselement 21 der Übertragungsvorrichtung 12 schließt sich eine Tropfenkammer 27 an, die aus einem transparenten Kunststoffkörper besteht. Die Tropfenkammer 27 ist an ihrem anderen Ende fest mit dem Übertragungsschlauch 22 verbunden. Das schlauchseitige Kupplungselement 21 weit an seinem freien Ende, der Tropfenkammer gegenüberliegend, einen Luer-Lock-Kupplungsring 28 auf, an den sich eine Einsteckhülse 29 mit einem Außenkonus anschließt. Dem Kupplungselement 21 der Übertragungsvorrichtung 12 benachbart ist eine Schlauchklemme 24 auf dem Schlauch 22 sitzend vorgesehen. Sie dient zum Einstellen des freien Durchflußquerschnittes des Schlauches 22.

Bei dem in den Fign. 1 und 2 gezeigten Infusionsset sind die Entnahmevorrichtung 15 und die Übertragungsvorrichtung bereits zusammengesteckt in der Umverpackung befindlich. Das Kupplungselement 20 der Entnahmevorrichtung besteht aus einem hülsenartigen Grundkörper 31, der eine in Längsrichtung verlaufende Einsteckbohrung 32 mit einem Innenkonus aufweist. An ihrem erweiterten Ende bildet die Einsteckbohrung 32 eine Einstecköffnung 33. An der Außenseite des Grundkörpers 31 im Bereich der Einstecköffnung 33 sind Gewindestege 34 angeordnet, die mit Gewindenuten an der Innenumfangsseite des Kupplungsringes 28 des schlauchseitigen Kupplungselementes 21 eine Luer-Lock Kupplung bilden und klemmend zusammengeschraubt werden können.

An dem der Einstecköffnung 33 gegenüberliegenden Ende 37 weist das schlauchseitige Kupplungselement 21 eine Öffnung 37 mit einem stiftartigen axial angeordneten Abbrechteil 38 auf, das von der Öffnung 37 der Einsteckbohrung 32 axial emporragt und einstückig an dem Öffnungsrand der Öffnung 37 mit einer dünnen umlaufenden Filmhaut 39 verbunden ist. Das Abbrechteil 38 kann mit geringem Kraftaufwand von dem Grundkörper 31 abgebrochen werden, so daß die der Einstecköffnung 33 gegenüberliegende Öffnung 37 geöffnet wird.

Der Infusionsbeutel 11 weist an seinem am tiefsten gelegenen Eckbereich zur Entnahme der Infusionslösung das von dem Infusionsbeutel 11 abgehende Schlauchstück 41 auf, das mit seinem einen Ende auf einen Öffnungsstutzen des Infusionsbeutels 11 aufgesteckt und mit seinem anderen Ende auf den Außenumfang des Grundkörpers 31 bis zu einem Anschlagring 35 auf ungefähr halber Länge des Grundkörpers 31 aufgeschoben ist. Das Abbrechteil 38 ragt in das Schlauchstück 41 hinein, das innen vier radiale, nach innen ragende Haltestege 42 aufweist.

In Fig. 2 sind die Kupplungselemente 20,21 im nicht zusammengesteckten Zustand vor der Sterilisierung und dem Verpacken dargestellt. Bei dem in Fig. 1 gezeigten zum Einsatz vorbereiteten Infusionsset 10 sind die Kupplungselemente 20,21 jedoch bereits zusammengesteckt. Dabei wird die den Außenkonus aufweisende Einsteckhülse 29 in den Innenkonus des Grundkörpers 31 eingesteckt und verklemmt und wird gleichzeitig der Luer-Lock-Kupplungsring auf das Gewinde 34 aufgesteckt und durch eine kleine Drehung verschraubt und befestigt.

Zum Einsatz des Infusionssets 10 wird zunächst die Umverpackung 13 geöffnet, indem sie an einem der beiden sich gegenüberliegenden zick-zack-förmigen Schweißränder eingerissen und geöffnet wird. Nach dem Aufhängen des Infusionsbeutels 11 und Applizieren einer Infusionsnadel an das Kupplungsteil 23 des Übertragungsschlauches 22 und ggf. Einlegen des Übertragungsschlauches 22 in eine Infusionspumpe wird die Schlauchklemme 24 geschlossen. Anschließend wird die durch das Abbrechteil 38 noch verschlossene Öffnung 37 durch den Bediener geöffnet, indem das flexible und transparente Schlauchstück 41 ergriffen und zusammengedrückt wird, so daß das Abbrechteil 38 ergriffen und unter Auftrennung der zirkulären Filmhaut 39 von dem Grundkörper 31 abgebrochen werden kann. Dabei wird das Abbrechteil 38 geringfügig axial in Richtung Infusionsbeutel 11 verschoben und in dieser neuen Position von den radialen Haltestegen 42 an der Schlauchinnenseite festgehalten, so daß das Abbrechteil 38 die Öffnung 37 nicht wieder verschließen oder verstopfen kann.

In dem in den Fign. 3 und 4 gezeigten zweiten Ausführungsbeispiel eines Infusionssets 10' ist das Kupplungselement 50 der Entnahmevorrichtung 15' anders ausgebildet als im ersten Ausführungsbeispiel der Fign. 1 und 2. Die Übertragungsvorrichtung 12 ist die gleiche, wie die des ersten Ausführungsbeispiels. Die Übertragungsvorrichtung 12 ist hier jedoch noch nicht mit der Entnahmevorrichtung 15' zusammengesteckt, sondern sie werden erst nach Öffnung der Umverpackung 13 zusammengesteckt.

Das Kupplungselement 50 der Entnahmevorrichtung ist als selbstschließendes Ventil ausgebildet. Es besteht aus zwei axial fest zusammengesteckten Teilen, nämlich aus dem Kupplungsteil 52 und dem Ventilteil 53. Das hülsenartige Kupplungsteil 52 hat einen Innenkonus 51 und weist an seiner Einstecköffnung 33 außenseitig umlaufend Gewindestege 34 für eine Luer-Lock-Kupplung auf. Auf die Einstecköffnung 33 ist eine Schutzkappe 66 auf das Kupplungsteil 52 aufgesteckt, um die Einstecköffnung 33 zu verschließen und gegen Verunreinigung zu schützen. Die Verschlußkappe 66 dient unzerstört als Siegel und wird beim Abziehen zerstört. An seinem anderen Ende weist das Kupplungsteil 52 eine Muffe 55 auf, die in eine Muffe 56 des Ventilteils 53 gesteckt ist.

In dem durch die zusammengesteckten Muffen 55,56 gebildeten ringförmigen Raum ist ein gewölbter Ventilteller 59 derart angeordnet, daß sein Rand in einer geschlossenen Ventilstellung auf den inneren Schultern der Steckmuffe 55 des Kupplungsteils 52 aufliegt. Die konvexe Seite des Ventiltellers 59 zeigt in Richtung der Einstecköffnung 33, also entgegen der Ausströmungsrichtung der Infusionslösung. Der Ventilteller 59 wird durch ein Stützkreuz 60 gehalten, dessen Kreuzarmenden an der Schulter der Muffe 56 des Ventilteils 53 abgestützt sind, und dessen Zentrum den Ventilteller 59 in der Mitte seiner konkaven Seite abstützt. Dadurch wird der Rand des Ventiltellers 59 auf die Schultern der Steckmuffe 55 des Kupplungsteils 52 gedrückt.

An der konvexen Seite des Ventiltellers 59 ist ein U-förmiger Rahmen 61 vorgesehen, wobei die freien Enden seiner Schenkel auf dem Randbereich des Ventiltellers 59 angeordnet sind, so daß der Rand des Ventiltellers 59 von den Rahmenschenkeln axial von der Schulter der Steckmuffe 55 abgehoben wird, wenn die Basis des Rahmens 61 axial in Einsteckrichtung gedrückt wird.

Am beutelseitigen Ende des Ventilteils 53 ist eine axiale Steckhülse 63 angeordnet, auf die das von dem Infusionsbeutel 11 kommende Schlauchstück 41 aufgesteckt und mit einer Überwurfmutter 64 auf der Steckhülse 63 festgeklemmt werden kann.

Beim Einsatz des Infusionssets 10' wird nach Öffnen der Umverpackung 13 zunächst die Schutzkappe 66 von dem beutelseitigen Kupplungselement 50 abgenommen. Nach dem Verschließen des Übertragungsschlauches 22 durch entsprechendes Betätigen der Schlauchklemme 24 wird das schlauchseitige Kupplungselement 21 in das beutelseitige Kupplungselement 50 eingesteckt, wobei der Kupplungsring 28 an den Gewindestegen 34 festgeschraubt wird. Dabei dringt die Einsteckhülse 29 des schlauchseitigen Kupplungsteils 21 so tief in das Kupplungsteil 52 ein, daß sie den Rahmen 61 axial verschiebt, so daß der Randbereich des Ventiltellers 59 von der umlaufenden Schulter der Muffe 55 abgehoben wird. Dadurch ist die Verbindung zwischen dem Infusionsbeutel 11 und dem Übertragungsschlauch 22 für die Infusionslösung geöffnet. Die Infusionslösung kann um den Rand des Ventiltellers 59 herum von dem Ventilteil 53 in das schlauchseitige Kupplungselement 21 fließen, von wo aus die Infusionslösung nach Durchlaufen der Tropfenkammer 27 schließlich über den Übertragungsschlauch 22 zum Patienten fließen kann. Wird das schlauchseitige Kupplungselement 21 abgezogen, kehrt der Ventilteller 59 wieder in die absperrende Ausgangslage zurück, so daß das Ventil wieder geschlossen ist und ein Nachlaufen oder -tropfen von Infusionslösung ausgeschlossen ist. Diese automatisch öffnende und schließende Kupplung eignet sich insbesondere für Cytostatika-Lösungen.

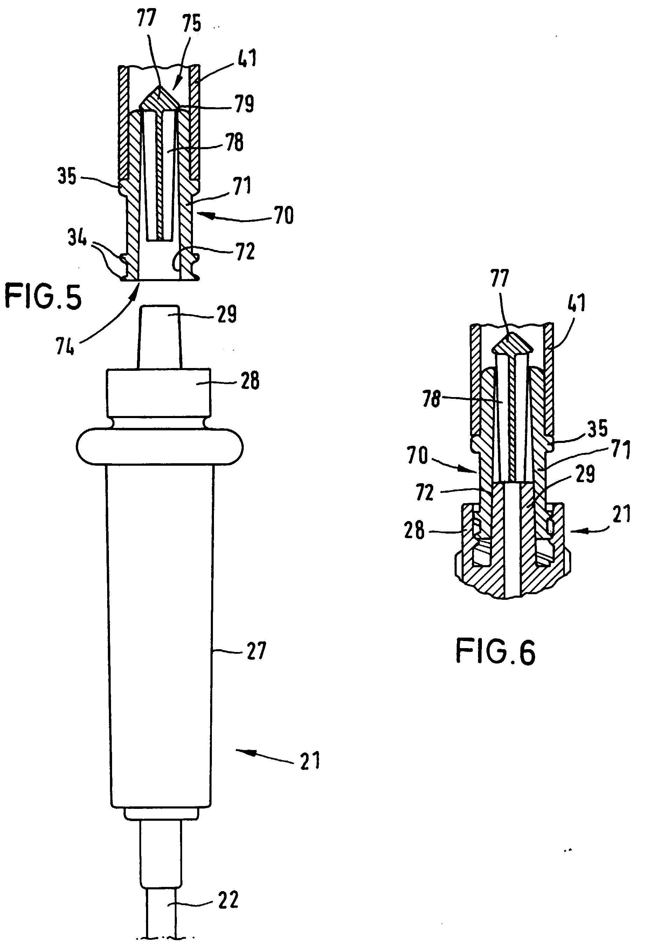

In den Fign. 5 und 6 ist eine dritte Ausführungsform von Kupplungselementen eines Infusionssets dargestellt. Das Kupplungselement 21 der Übertragungsvorrichtung 15 ist identisch mit dem des ersten und zweiten Ausführungsbeispiels. Das Kupplungselement 70 ist ein länglicher Rohrkörper 71, das mit einem Innenkegel 72 versehen ist. Das eine Ende des Innenkegels 72 bildet die Einstecköffnung 74 für die konische Einsteckhülse 29 des schlauchseitigen Kupplungselementes 21. Auf den außenseitig zylindrischen Rohrkörper 71 ist der Einstecköffnung 74 gegenüberliegend das Schlauchstück 41 bis zum Anschlagring 35 aufgesteckt. Auch dieses Kupplungselement 70 bildet zusammen mit dem Kupplungsring 28 des schlauchseitigen Kupplungselementes 21 eine Luer-Lock-Kupplung.

Das Kupplungselement 70 der Entnahmevorrichtung ist mit einem Brechsiegel 75 versehen, das aus einem Sperrkörper 77 und einem sich daran anschließenden Schaft 78 besteht. Der Sperrkörper 77 ist an dem dem Einsteckende 74 abgewandten Ende des beutelseitigen Kupplungselementes 70 angeordnet. Der Sperrkörper 77 steht über dieses Ende hinaus vor, während der Schaft 78 sich in den Innenkegel 72 hinein ersteckt.

Der Sperrkörper 77 hat generell die Gestalt eines Kegels, wobei der Schaft 78 sich an die Basisfläche des Kegels anschließt. Der Umfang der Basisfläche des Kegels ist über eine aus einer Filmhaut 79 bestehenden Sollbruchlinie mit dem Rohrkörper 71 verbunden. Die Sollbruchlinie bildet einen geschlossenen Ring, der zusammen mit dem Sperrkörper 77 den Innenkegel 72 flüssigkeitsdicht verschließt und die einzige Verbindung des Brechsiegels 79 mit dem Rohrkörper 71 darstellt. Der Rohrkörper 71 ist einstückig mit dem Brechsiegel 75 und aus Kunststoff bestehend ausgebildet.

In Fig. 6 ist der Zustand dargestellt, in dem die Kupplungselemente 21,70 zusammengesteckt sind. Die Einsteckhülse 29 des schlauchseitigen Kupplungselements 21 hat beim Eindringen in den Innenkegel 72 des beutelseitigen Kupplungselementes 70 den Schaft 78 zusammen mit dem Sperrkörper 77 in das Schlauchstück 41 hineingedrückt, wobei die zirkuläre Filmhaut 79 gebrochen ist. Dadurch kann der Sperrkörper 77 von dem Ansatz des Rohrkörpers 71 weggedrückt und abgelöst werden und die Kupplung 21,70 ist geöffnet für die Infusionslösung.

Alle Teile des Infusionssets sind aus Kunststoff hergestellt.

Neben flexiblen Beuteln ist das Infusionsset auch mit anderen Behältern, beispielsweise Blowfill-Seal-Containern (Bottlepack-Behälter) anwendbar.

## Patentansprüche

1. Infusionsset mit einem Container (11), der eine Entnahmevorrichtung (15,15') aufweist, und einer an die Entnahmevorrichtung (15,15') anschließbaren Übertragungsvorrichtung (12) mit einer Tropfkammer (27) und einem Übertragungsschlauch (12),
**dadurch gekennzeichnet,**
daß die Entnahmevorrichtung (15,15') des Containers (11) und die Übertragungsvorrichtung (12) zusammenpassende Kupplungselemente (20,21,50,70) aufweisen, und daß eines dieser Kupplungselemente (20,50,70) einen Verschluß enthält, der beim Zusammenstecken mit dem anderen Kupplungselement (21) automatisch öffnet oder nach dem Zusammenstecken durch externe Betätigung manuell öffnet.

2. Infusionsset nach Anspruch 1, dadurch gekennzeichnet, daß eines der Kupplungselemente (20,50,70) einen Innenkonus (35,51,72) und das andere (21) einen dazu passenden Außenkonus aufweist.

3. Infusionsset nach Anspruch 1 oder 2, dadurch gekennzeichnet, daß das Kupplungselement (21) der Übertragungsvorrichtung (12) einteilig mit der Tropfkammer (23) ausgebildet ist.

4. Infusionsset nach einem der Ansprüche 13, dadurch gekennzeichnet, daß die Entnahmevorrichtung (15) an einem von dem Container (11) abgehenden Schlauchstück (41) angebracht ist.

5. Infusionsset nach einem der Ansprüche 1-4, dadurch gekennzeichnet, daß der Verschluß als selbstschließendes Ventil (50) ausgebildet ist.

6. Infusionsset nach einem der Ansprüche 1-4, dadurch gekennzeichnet, daß der Verschluß ein Abbrechteil (38,75) ist.

7. Infusionsset nach Anspruch 6, dadurch gekennzeichnet, daß das Abbrechteil (38) in einen Schlauch (41) hineinragt und unter Verformung des Schlauchs (41) abgebrochen werden kann.

8. Infusionsset nach Anspruch 6 oder 7, dadurch gekennzeichnet, daß das Abbrechteil (75) beim Zusammenstecken der Kupplungselemente (70) abbricht.

9. Infusionsset nach einem der Ansprüche 1-8, dadurch gekennzeichnet, daß das den Verschluß aufweisende Kupplungselement (20,50,70) an der Entnahmevorrichtung (15,15') des Containers (11) vorgesehen ist.

10. Infusionsset nach Anspruch 3, dadurch gekennzeichnet, daß der Innenkonus (32,72) an dem Kupplungselement (20,50,70) des Containers (11) und der Außenkonus (29,51) an der Tropfkammer (27) vorgesehen ist.

11. Infusionsset nach einem der Ansprüche 1-10, dadurch gekennzeichnet, daß der Container (11) und die Übertragungsvorrichtung (12) steril in einer gemeinsamen Umverpackung (13) enthalten sind.

12. Kupplungselement für den Anschluß an eine Entnahmevorrichtung (15,15') eines eine Infusionslösung enthaltenden Containers mit den Merkmalen eines der Ansprüche 1-11.
